# EUROPÄISCHE PATENTSCHRIFT

(11) **EP 2 663 347 B1**
(45) Veröffentlichungstag und Bekanntmachung des Hinweises auf die Patenterteilung: **19.08.2015**
(21) Anmeldenummer: 12701825.7
(22) Anmeldetag: 10.01.2012
(51) Int. Cl.: A61M 1/26

(54) **BLUTBEHANDLUNGSEINHEIT FÜR EINE EXTRAKORPORALE BLUTBEHANDLUNGSVORRICHTUNG**
BLOOD TREATMENT UNIT FOR AN EXTRACORPOREAL BLOOD TREATMENT DEVICE
UNITÉ DE TRAITEMENT DU SANG DESTINÉE À UN DISPOSITIF EXTRACORPOREL DE TRAITEMENT DU SANG

(30) Priorität: 11.01.2011 DE 102011008329
(43) Veröffentlichungstag der Anmeldung: 20.11.2013
(73) Patentinhaber: Fresenius Medical Care Deutschland GmbH, 61352 Bad Homburg v.d.H. (DE)
(72) Erfinder: HEIDE, Alexander, 65817 Eppstein (DE); WIKTOR, Christoph, 63571 Gelnhausen (DE); PETERS, Arne, 61352 Bad Homburg (DE)
(74) Vertreter: Oppermann, Frank
(86) Internationale Anmeldenummer: PCT/EP2012/000073
(87) Internationale Veröffentlichungsnummer: WO 2012/095294

(56) Entgegenhaltungen:
- EP-A1- 0 576 677
- WO-A2-93/05828
- US-A- 3 771 658

## Beschreibung

Die Erfindung betrifft eine Blutbehandlungseinheit für eine extrakorporale Blutbehandlungsvorrichtung mit einem extrakorporalen Blutkreislauf, der eine arterielle Blutleitung zum Zuführen von Blut und eine venöse Blutleitung zum Abführen von Blut aufweist. Darüber hinaus betrifft die Erfindung eine extrakorporale Blutbehandlungsvorrichtung mit der erfindungsgemäßen Blutbehandlungseinheit.

Zur Entfernung von harnpflichtigen Substanzen und zum Flüssigkeitsentzug werden verschiedene Verfahren zur extrakorporalen Blutbehandlung eingesetzt. Bei der Hämodialyse wird das Blut des Patienten außerhalb des Körpers in einem Dialysator gereinigt. Der Dialysator weist eine Blutkammer und eine Dialysierflüssigkeitskammer auf, die durch eine semipermeable Membran getrennt sind. Während der Behandlung strömt Blut des Patienten durch die Blutkammer, während die Dialysierflüssigkeitskammer von Dialysierflüssigkeit durchströmt wird, um das Blut effektiv zu reinigen.

Bei der Hämodialyse (HD) wird der Transport der kleinmolekularen Substanzen durch die semipermeable Membran des Dialysators im Wesentlichen durch die Konzentrationsunterschiede (Diffusion) zwischen dem Blut und der Dialysierflüssigkeit bestimmt. Bei der Hämofiltration (HF) wird mittels eines an der semipermeablen Membran angelegten Druckgradienten (Transmembrandruck) dem Blut über die Membran Flüssigkeit entzogen (Ultrafiltration). Als Hämodiafiltration wird ein Verfahren zur extrakorporalen Blutbehandlung bezeichnet, bei dem sowohl eine Hämodialyse als auch Hämofiltration durchgeführt wird.

Während der extrakorporalen Blutbehandlung findet der Stoffaustausch im Dialysator statt. Die am häufigsten eingesetzte Bauform eines Dialysators ist der sogenannte Kapillardialysator, der aus einem Gehäuse besteht, in dem eine große Anzahl von Hohlfasern parallel angeordnet sind. In den Hohlfasern strömt das Blut, während die Dialysierflüssigkeit entlang des Hohlfaserbündels strömt. Zur Erhöhung der Effektivität des Stoffübergangs fließen Blut und Dialysierflüssigkeit im Gegenstrom. Eine extrakorporale Blutbehandlungsvorrichtung für die Hämodialyse, Hämofiltration und Hämodiafiltration wird nachfolgend als Dialysevorrichtung bezeichnet.

Die bekannten Dialysevorrichtungen verfügen über peristaltische Pumpen, mit denen das Blut im extrakorporalen Blutkreislauf durch den Dialysator gefördert wird.

Aus der DE 39 23 692 A1 ist ein medizinisches Gerät für den Stoff- und/oder Wärmeaustausch zwischen einer Flüssigkeit und einem Behandlungsmedium bekannt, bei dem die das Behandlungsmedium fördernde Pumpe zusammen mit der Blutbehandlungseinheit in einem langgestreckten Gehäuse hintereinander angeordnet sind. Das rohrförmige Gehäuse umfasst einen Pumpenraum mit einem Pumpenrad und einen Raum, in dem sich ein Hohlfaserbündel befindet, dessen Hohlfasern sich in Längsrichtung des rohrförmigen Gehäuses erstrecken. Das rotierende Pumpenrad fördert das Behandlungsmedium durch die Hohlfasern des Hohlfaserbündels, während das Hohlfaserbündel von einer Flüssigkeit umströmt wird.

Auf dem Gebiet der Oxygenierung von Blut finden zur Förderung des Bluts Impellerpumpen Verwendung. Bei der Blutoxygenierung wird das in einem extrakorporalen Blutkreislauf zirkulierende Blut über eine selektive Membran mit Sauerstoff in Kontakt gebracht. Als selektive Membranen werden auch Hohlfaserbündel eingesetzt. Im Gegensatz zur Dialyse werden die Hohlfasern aber nicht von dem Blut, sondern von dem Behandlungsmedium (Sauerstoff) durchströmt, während das Blut entlang des Hohlfaserbündels strömt.

Es sind verschiedene Bauarten von Impellerpumpen bekannt. Die Impellerpumpen zeichnen sich insbesondere dadurch aus, dass sie über einen Propeller verfügen, der von einem ring- oder rohrförmigen Gehäuse umschlossen wird.

Die WO 93/05828 und die EP 1 930 034 A1 beschreiben Impellerpumpen für die Blutoxygenierung. Beide Impellerpumpen zeichnen sich dadurch aus, dass das Blut von dem Propeller der Impellerpumpe entlang des Hohlfaserbündels gefördert wird, während der Sauerstoff durch die Hohlfasern strömt.

Die EP 0 576 677 A1 beschreibt eine Blutbehandlungseinheit, die ein zylindrisches Gehäuse aufweist, in dem eine Impellerpumpe P angeordnet ist. Die Blutbehandlungseinheit verfügt über eine zylindrische Filtereinheit, die aus einer Vielzahl von Hohlfasem besteht. Das Gas strömt durch die Lumen der sich in axialer Richtung erstreckenden Hohlfasern der zylindrischen Hohlfaser-Filtereinheit, während die Hohlfasern von Blut umströmt werden, so dass ein Stoffaustausch zwischen Gas und Blut stattfindet. Dabei werden Gas und Blut aber nicht von einer den Impeller der Impellerpumpe umschließenden "Trennwand" getrennt. Vielmehrt strömt das Blut von Innen nach Außen in radialer Richtung durch die zwischen den Hohlfasern befindlichen Zwischenräume der Hohlfaser-Filtereinheit.

Der Erfindung liegt die Aufgabe zugrunde, eine Alternative zu den Dialysatoren und Blutpumpen der bekannten extrakorporalen Dialysevorrichtungen anzugeben.

Die Lösung dieser Aufgabe erfolgt erfindungsgemäß mit den Merkmalen des Patentanspruchs 1, Vorteilhafte Ausführungsformen der Erfindung sind Gegenstand der Unteransprüche.

Die erfindungsgemäße Blutbehandlungseinheit ist für eine extrakorporale Dialysevorrichtung bestimmt. Sie zeichnet sich durch einen besonders kompakten Aufbau aus, sodass sie auch für tragbare Dialysegeräte geeignet ist. Insgesamt kann der extrakorporale Blutkreislauf der Dialysevorrichtung mit der erfindungsgemäßen Blutbehandlungseinheit erheblich verkleinert werden.

Das Gehäuse der erfindungsgemäßen Blutbehandlungseinheit nimmt sowohl die Blutpumpe als auch die semipermeable Membran des Dialysators der extrakorporalen Dialysevorrichtung auf. Obwohl nachfolgend von einem gemeinsamen Gehäuse für Blutpumpe und semipermeable Membran die Rede ist, kann das Gehäuse auch aus mehreren Gehäuseteilen, beispielsweise zwei Gehäusehälften bestehen, die miteinander lösbar verbunden sind.

Bei der Blutpumpe handelt es sich um eine Impellerpumpe, wobei der Impeller in dem Gehäuse um eine Achse drehbar gelagert ist. Der Impeller wird von einer das Gehäuse in zwei Kammern unterteilenden Trennwand umschlossen. Da die semipermeable Membran zumindest einen Teil der Trennwand bildet, ist es möglich, dass ein Stoffaustausch zweischen dem Blut, das die erste Kammer durchströmt, und der die zweite Kammer des Gehäuses durchströmenden Dialysierflüssigkeit stattfinden kann.

Obwohl die erfindungsgemäße Blutbehandlungseinheit relativ kleine Abmessungen haben kann, erlaubt die Impellerpumpe, die mit einer hohen Drehzahl betrieben werden kann, eine effektive Dialysebehandlung. Das von der Impellerpumpe geförderte Blut durchströmt wegen der hohen Drehzahl der Pumpe mehrfach die erste Kammer des Gehäuses, bevor das Blut die Kammer wieder verlässt. Dadurch ist die Blutströmung an der Grenzfläche der Membran besonders effektiv.

Bei der erfindungsgemäßen Blutbehandlungseinheit ist also für die Baugröße nicht nur die semipermeable Membran (Dialysator) entscheidend, sondern die Drehzahl und der Durchmesser des Impellers der Impellerpumpe. Da Impellerpumpen im Allgemeinen mit hoher Drehzahl betrieben werden und verhältnismäßig geringe Abmessungen haben, kann insgesamt eine Blutbehandlungseinheit mit einem kompakten Aufbau geschaffen werden.

Die erfindungsgemäße Blutbehandlungseinheit kann nicht nur für die Hämodialyse, sondern auch die Hämofiltration Verwendung finden. Für die Hämofiltration wird die zweite Kammer des Gehäuses aber nicht von Dialysierflüssigkeit durchströmt. Selbstverständlich kann die erfindungsgemäße Blutbehandlungseinheit auch für die Hämodiafiltration verwendet werden.

Die semipermeable Membran der erfindungsgemäßen Blutbehandlungseinheit kann aus den bekannten Filtermaterialien für Dialysemembranen bestehen. Typische semipermeable Filtermaterialien basieren auf Polysulfon oder Zelluloseacetat. Es ist aber auch möglich PTFE-Membranen, Membranen aus Polykarbonat oder sogar Keramikmembranen einzusetzen. Auch können in Mikrostrukturtechnik durch Ätzprozesse hergestellte Membranen aus Halbleitermaterialien verwendet werden.

Das Gehäuse weist einen von der ersten Kammer abgehenden Auslasskanal zum Abführen des Bluts auf, der sich in radialer Richtung erstreckt.

Ein besonders kompakter Aufbau ergibt sich daraus, dass der Auslasskanal zum Abführen von Blut die semipermeable Membran in radialer Richtung durchdringt, wobei die Effektivität dann am höchsten ist, wenn die semipermeable Membran den Impeller bis auf den Bereich des Auslasskanals vollständig umschließt.

Bei einer bevorzugten Ausführungsform der erfindungsgemäßen Blutbehandlungseinheit weist das Gehäuse einen zu der ersten Kammer führenden Einlasskanal zum Zuführen von Blut auf, der sich in Richtung der Drehachse des Impellers erstreckt. Folglich wird das Blut der ersten Kammer in axialer Richtung zugeführt. Folglich wird das Blut in axialer Richtung zugeführt und mittels des rotierenden Impeller in Richtung der den Impeller umschließenden Membran gefördert, wobei das Blut, nachdem es in der ersten Kammer die Membran mehrfach umströmt hat, in radialer Richtung wieder aus der Kammer abgeführt wird.

Das Gehäuse weist vorzugsweise ein zu der zweiten Kammer führenden Einlasskanal zum Zuführen von Dialysierflüssigkeit und einen von der zweiten Kammer abgehenden Auslasskanal zum Abführen von Dialysierflüssigkeit auf. Damit kann die erfindungsgemäße Blutbehandlungseinheit nicht nur für die Hämofiltration, sondern auch Hämodialyse Verwendung finden.

Die zweite Kammer des Gehäuses ist vorzugsweise als Ringraum ausgebildet, der die erste Kammer des Gehäuses vorzugsweise vollständig umschließt, wobei die semipermeable Membran die erste und zweite Kammer voneinander trennen. Wenn die semipermeable Membran nur Teile der das Gehäuse in die erste und zweite Kammer unterteilenden Trennwand bilden sollte, umschließt die zweite Kammer vorzugsweise den Bereich der Trennwand, an dem sich die semipermeable Membran befindet. Vorzugsweise sind die Kammern und die Membran derart ausgebildet und angeordnet, dass die gesamte Membranfläche von beiden Seiten angeströmt wird.

Bei einer besonders bevorzugten Ausführungsform ist die semipermeable Membran an der Oberfläche, die der ersten Kammer zugewandt ist, mit Rillen versehen. Die Rillen können als Längs- und/oder Querrillen ausgebildet sein. Mit den Längs- und/oder Querrillen können die roten Blutkörperchen sich nach dem Fahraeus-Lindqvist-Effekt in der Mitte der Strömungskanäle ausrichten und einen erhöhten Plasmaanteil an der Filteroberfläche ausbilden.

Die erfindungsgemäße extrakorporale Dialysevorrichtung verfügt über die erfindungsgemäße Blutbehandlungseinheit. Sie weist darüber hinaus eine arterielle Blutleitung zum Zuführen von Blut in die erste Kammer und eine venöse Blutleitung zum Abführen von Blut aus der ersten Kammer der Blutbehandlungseinheit auf. Darüber hinaus kann die extrakorporale Dialysevorrichtung eine Dialysierflüssigkeitszuführleitung und Dialysierflüssigkeitsabführleitung zum Zu- bzw. Abführen von Dialysierflüssigkeit in bzw. aus der zweiten Kammer der Blutbehandlungseinheit aufweisen.

Von Vorteil ist, dass sich die Ultrafiltrationsrate auf einfache Weise dadurch steuern lässt, dass der Blutfluss in der Blutabführleitung gedrosselt wird. Daher sieht eine bevorzugte Ausführungsform der extrakorporalen Dialysevorrichtung in der Blutabführleitung angeordnete Mittel zum Drosseln des Blutflusses vor. Entscheidend ist der Aufbau einer transmembranen Druckdifferenz. Eine transmembrane Druckdifferenz kann beispielsweise durch Drosselelemente auf der Blutseite aufgebaut werden, die durch Verringerung des Blutflusses einen Druckaufbau verursachen. Solche Mittel können beispielsweise ein steuerbares Ventil sein. Die Druckdifferenz kann auch durch eine zweite Pumpe aufgebaut werden, die der Pumprichtung des Impellers entgegen wirkt. Der Aufbau der Druckdifferenz kann auch durch Mittel auf der Dialysierflüssigkeitsseite erfolgen, beispielsweise durch eine zusätzliche Ultrafiltrationspumpe, die gegenüber dem Druck auf der Blutseite einen Unterdruck erzeugt.

Eine weitere besonders bevorzugte Ausführungsform der extrakorporalen Dialysevorrichtung weist eine Steuereinheit auf, die derart ausgebildet ist, dass die Blutpumpe und/oder die Dialysierflüssigkeitspumpe in einem Pulsmodus betrieben werden können. Der Pulsmodus zeichnet sich dadurch aus, dass die Blut- bzw. Dialysierflüssigkeitspumpe fortlaufend ein- und ausgeschaltet oder die Drehzahl der Pumpen fortlaufend verändert, d. h. erhöht und verringert wird. Der Betrieb der Blutpumpe im Pulsmodus kann die Diffusionsvorgänge an der Membran begünstigen. Auch der Betrieb der Dialysierflüssigkeitspumpe kann den Filtereffekt positiv beeinflussen, da die Poren des Filters mit den dialysatseitigen Druckpulsen zyklisch "gespült" werden.

Im Folgenden werden zwei Ausführungsbeispiele der Erfindung unter Bezugnahme auf die Zeichnungen näher erläutert.

Es zeigen:
- Fig. 1: ein erstes Ausführungsbeispiel der erfindungsgemäßen Blutbehandlungseinheit in stark vereinfachter schematischer Darstellung,
- Fig. 2: einen Schnitt entlang der Linie II-II von Fig. 1,
- Fig. 3: in stark vereinfachter schematischer Darstellung ein zweites Ausführungsbeispiel der erfindungsgemäßen Blutbehandlungseinheit,
- Fig. 4: einen Schnitt entlang der Linie IV-IV von Fig. 3 und
- Fig. 5: ein Ausführungsbeispiel einer extrakorporalen Dialysevorrichtung mit der erfindungsgemäßen Blutbehandlungseinheit in stark vereinfachter schematischer Darstellung.

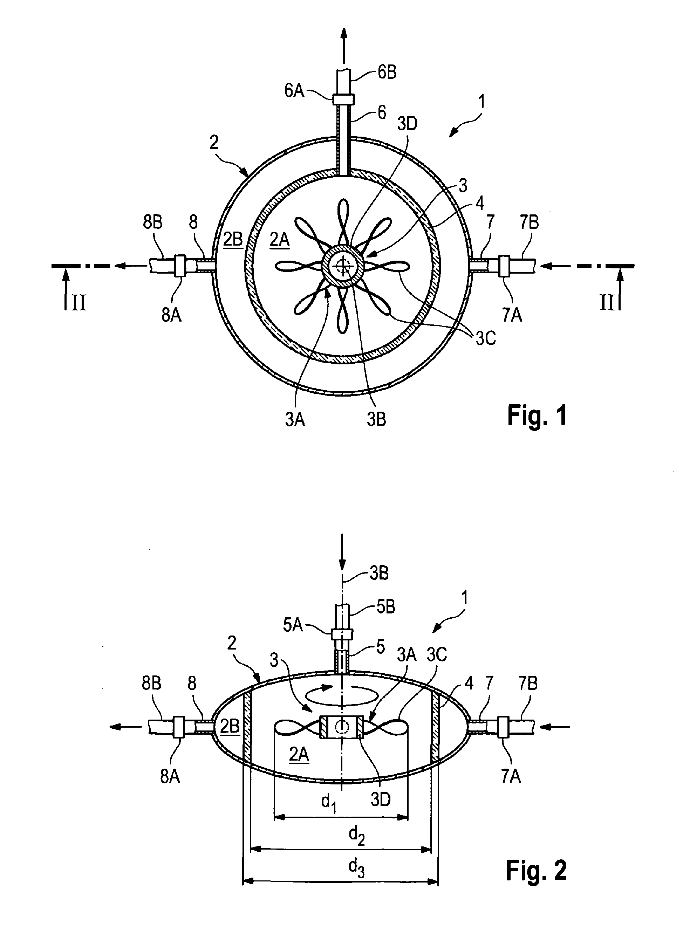

Die Figuren 1 und 2 zeigen einen Schnitt durch die erfindungsgemäße Blutbehandlungseinheit in stark vereinfachter schematischer Darstellung. Die Blutbehandlungseinheit 1 weist ein zylindrisches Gehäuse 2 auf, das aus zwei miteinander verschraubten Gehäusehälften bestehen kann. In dem zylindrischen Gehäuse 2 befindet sich eine Blutpumpe 3. Bei der Blutpumpe handelt es sich um eine Impellerpumpe 3. Die Impellerpumpe 3 weist einen Impeller 3A auf, der um eine Achse 3B in dem Gehäuse drehbar gelagert ist. Der Impeller 3A kann in dem Gehäuse 2 mechanisch, elektromagnetisch, hydrodynamisch oder hydrostatisch gelagert sein. Er weist mehrere Propeller-, Schaufel- oder Flügelspitzen 3C auf, die sich vom Zentrum nach außen erstrecken. Zum Antrieb des Impellers 3A ist eine Antriebseinheit 3D vorgesehen, die beispielsweise ein elektromagnetischer, pneumatischer oder mechanischer Antrieb sein kann. Der Außendurchmesser des Impellers 3A ist in mit d₁ bezeichnet.

Der Impeller 3A wird von einer semipermeablen Membran 4 umschlossen, die sich über einen Umfangswinkel von 360°C um den Impeller 3A erstreckt. Die Membran 4 hat einen Innendurchmesser d₂ und einen Außendurchmesser d₃. Die Dicke der Membran ist d₃ - d₂.

Der Innendurchmesser d₂ der semipermeablen Membran 4 und der Außendurchmesser d₁ des Impellers 3A sind so bemessen, dass zwischen dem Impeller und der Membran ein Zwischenraum verbleibt.

Die semipermeable Membran 4 teilt das zylindrische Gehäuse 2 in eine erste innere Kammer 2A und eine zweite äußere Kammer 2B auf. Daher bildet die semipermeable Membran 4 in dem Gehäuse 2 eine Trennwand zwischen den beiden Kammern 2A und 2B.

Das Gehäuse 2 weist einen axialen Einlasskanal 5 auf, der sich durch die Gehäusewand in Richtung der Drehachse 3B des Impellers 3A erstreckt. An dem Einlass des Einlasskanals 5 befindet sich ein Anschlussstück 5A zum Anschluss einer nur andeutungsweise dargestellten Blutzuführleitung 5B. Darüber hinaus weist das Gehäuse 2 einen radialen Auslasskanal 6 zum Abführen von Blut auf, der sich in radialer Richtung durch die Gehäusewandung erstreckt und die semipermeable Membran 4 durchdringt. Am Auslass des Auslasskanals 6 befindet sich ein Anschlussstück 6A zum Anschluss einer nur andeutungsweise dargestellten Blutabführleitung 6B.

Wenn der Impeller 3A rotiert, wird über den Einlasskanal 5 Blut in die erste Kammer 2A des Gehäuses in axialer Richtung angesaugt. Aufgrund der Rotation des Impellers 3A strömt das Blut in der ersten Kammer 2A mehrfach entlang des Innenumfangs der semipermeablen Membran 4, bevor das Blut über den Auslasskanal 6 die erste Kammer 2A wieder verlässt.

Die zweite Kammer 2B des Gehäuses 2 bildet einen Ringraum, der die Außenseite der semipermeablen Membran 4 vollständig umschließt.

Neben dem Ein- und Auslasskanal 5, 6 zum Zu- und Abführen von Blut ist in dem Gehäuse 2 ein Einlasskanal 7 zum Zuführen von Dialysierflüssigkeit in die zweite Kammer 2B und ein Auslasskanal 8 zum Abführen von Dialysierflüssigkeit aus der zweiten Kammer ausgebildet. Am Einlass des Einlasskanals 7 und dem Auslass des Auslasskanals 8 befindet sich jeweils ein Anschlussstück 7A bzw. 8A zum Anschluss einer nur andeutungsweise dargestellten Dialysierflüssigkeitszuführ- bzw. -Abführleitung 7B bzw. 8B. Einlass- und Auslasskanal 7, 8 zum Zu- bzw. Abführen von Dialysierflüssigkeit sind derart angeordnet, dass in dem Ringraum die Dialysierflüssigkeit entlang der Außenfläche der semipermeablen Membran 4 strömt.

Während des Betriebs der Blutbehandlungseinheit 1 strömen also Blut und Dialysierflüssigkeit entlang der Innen- bzw. Außenseite der semipermeablen Membran 4. Folglich wird die semipermeable Membran 4 von beiden Seiten umströmt. Dabei kann es für das Strömungsverhalten vorteilhaft sein, wenn die semipermeable Membran 6 an der Innenseite feine Längs- und/oder Querrillen aufweist, die eine Tiefe von ca. 50 µm haben können.

Die semipermeable Membran 4 kann eine Stärke von 1 bis 10 mm haben. Sie kann aus einer funktionellen Membranschicht und einer mechanisch stützenden Schicht bestehen, wobei die funktionelle Schicht die für die Therapie notwendige Filtrationswirkung leistet, die von den für die Therapie wichtigen Parametern, insbesondere von dem Siebkoeffizienten, der hydraulischen Permeabilität und der Biokompatibilität bestimmt wird. Die Funktionsschicht kann Dicken von 15 bis 100 µm haben.

Die Impellerpumpe 3 kann beispielsweise mit einer Drehzahl von ca. 7000 U/min betrieben werden. Der Außendurchmesser d₂ des Impellers liegt bei dem vorliegenden Ausführungsbeispiel bei 30 mm und die semipermeable Membran 4 hat bei dem Ausführungsbeispiel eine Stärke d₃- d₂ von 10 mm haben.

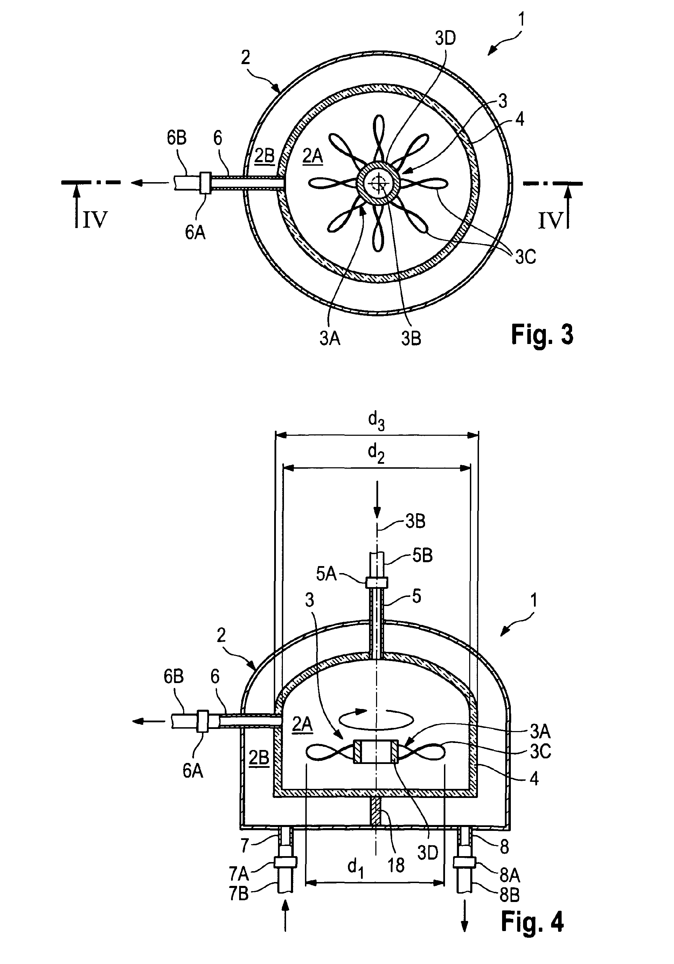

Die Figuren 3 und 4 zeigen in stark vereinfachter schematischer Darstellung ein zweites Ausführungsbeispiel der erfindungsgemäßen Blutbehandlungseinheit. Das zweite Ausführungsbeispiel unterscheidet sich von der ersten Ausführungsform nur durch die Gehäuseform. Daher werden die einander entsprechenden Teile auch mit den gleichen Bezugszeichen bezeichnet.

Auch bei der alternativen Ausführungsform wird das Blut über den Einlasskanal 5 in axialer Richtung durch den rotierenden Impeller 3 in die erste Kammer 2A des Gehäuses 2 angesaugt, um das Gehäuse dann wieder über den Auslasskanal 6 in radialer Richtung zu verlassen. Auch die zweite Kammer 2B des Gehäuses 2 wird wieder von Dialysierflüssigkeit durchströmt, das über den Einlasskanal 7 zu- und den Auslasskanal 8 abgeführt wird. Eine zwischen dem Einlasskanal 7 und dem Auslasskanal 8 angeordnete Trennwand 18, die sich zwischen der Innenwand des Gehäuses 2 und der Außenwand der Membran 4 erstreckt, verhindert einen Kurzschlussstrom des Dialysats zwischen den Anschlüssen. Bei dem alternativen Ausführungsbeispiel wird nicht nur die äußere Umfangsfläche des Impellers 3A von der semipermeablen Membran 4 und der zweiten Kammer 2B des Gehäuses umschlossen, sondern die semipermeable Membran 4 umschließt den Impeller 3 von allen Seiten. Die zweite Kammer 2B des Gehäuses 2 ist bei dem alternativen Ausführungsbeispiel als ein die semipermeable Membran 4 von allen Seiten umschließender Raum ausgebildet. Dadurch wird erreicht, dass die wirksame Fläche der Membran größer als bei dem ersten Ausführungsbeispiel ist.

Die erfindungsgemäße Blutbehandlungseinheit 1 findet bei einer extrakorporalen Dialysevorrichtung Verwendung, die nachfolgend beschrieben wird. Da die Dialysevorrichtungen zum Stand der Technik gehören, werden nur die wesentlichen Komponenten der Vorrichtung beschrieben.

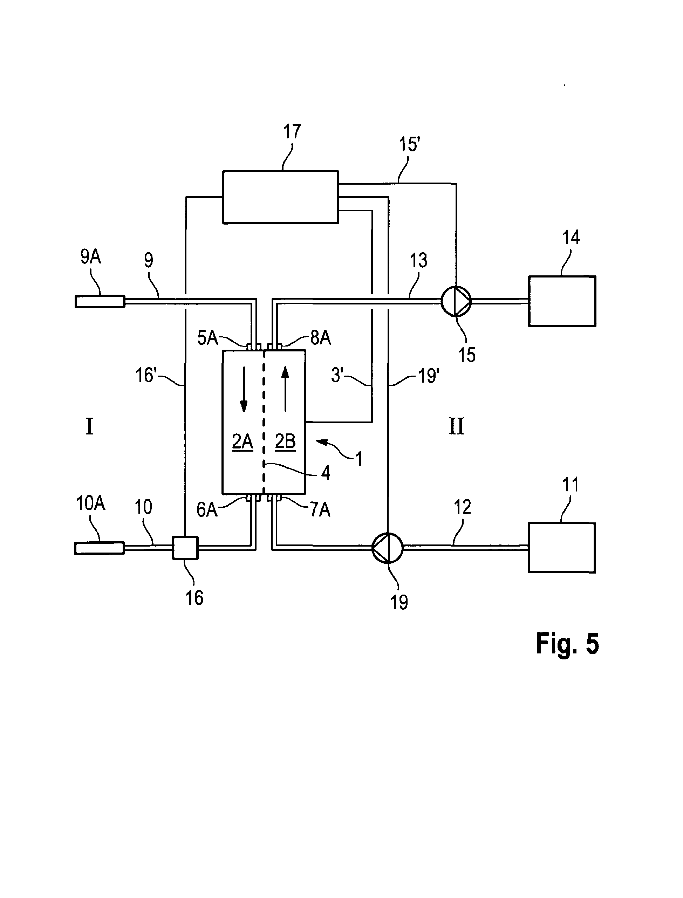

Fig. 5 zeigt in stark vereinfachter schematischer Darstellung die wesentlichen Komponenten der extrakorporalen Dialysevorrichtung, die über die erfindungsgemäße Blutbehandlungseinheit 1 verfügt. Die Dialysevorrichtung weist eine arterielle Blutleitung 9 und eine venöse Blutleitung 10 auf. An dem einen Ende der arteriellen Blutleitung befindet sich ein arterieller Patientenanschluss 9A (Kanüle), während das andere Ende der arteriellen Blutleitung 9 an das Anschlussstück 5A am Einlasskanal 5 der ersten Kammer 2A der Blutbehandlungseinheit 1 angeschlossen ist. Die venöse Blutleitung 10 weist an dem einen Ende einen venösen Patientenanschluss 10A (Kanüle) auf, während das andere Ende der venösen Blutleitung 10 an das Anschlussstück 6A am Auslasskanal 6 der ersten Kammer 2A der Blutbehandlungseinheit 1 angeschlossen ist (Figuren 1 bis 4). Von einer Dialysierflüssigkeitsquelle 11 führt eine Dialysierflüssigkeitszuführleitung 12 zu dem Anschlussstück 7A am Einlasskanal 7 der zweiten Kammer 2B der Blutbehandlungseinheit 1. Von dem Anschlussstück 8A des Auslasskanals 8 der zweiten Kammer 2B der Blutbehandlungseinheit geht eine Dialysierflüssigkeitsabführleitung 13 ab, die zu einem Ablauf 14 führt. In der Dialysierflüssigkeitsabführleitung 13 ist eine als Ultrafiltrationspumpe wirkende Pumpe 15 angeordnet, während sich in der arteriellen Blutleitung 10 Mittel 16 zum Drosseln des Blutflusses befinden. Bei diesen Mitteln kann es sich um eine elektromagnetisch betätigbare Drossel 16 handeln. Die Dialysierflüssigkeit wird mit einer Dialysierflüssigkeitspumpe 19 gefördert, die stromauf der Blutbehandlungseinheit 1 in der Dialysierflüssigkeitszuführleitung 12 angeordnet ist.

Darüber hinaus verfügt die Dialysevorrichtung über eine zentrale Steuereinheit 17, die über Steuerleitungen 3', 15', 16', 19' mit der in Fig. 4 nicht dargestellten Impellerpumpe 3 der Blutbehandlungseinheit 1, der Ultrafiltrationspumpe 15, der elektromagnetisch betätigbaren Drossel 16 und der Dialysierflüssigkeitspumpe 19 verbunden sind.

Während der Dialyse fördert die Impellerpumpe 3 der Blutbehandlungseinheit 1 Blut des Patienten in dem extrakorporalen Blutkreislauf I durch die erste Kammer 2A der Blutbehandlungseinheit 1, während die Dialysierflüssigkeitspumpe 19 Dialysierflüssigkeit in dem Dialysierflüssigkeitskreislauf II im Gegenstrom durch die zweite Kammer 2B der Blutbehandlungseinheit fördert, sodass ein Stoffaustausch zwischen Blut und Dialysierflüssigkeit stattfinden kann.

Durch Veränderung der Drehzahl der Impellerpumpe und des Transmembrandrucks an der semipermeablen Membran 4 der Blutbehandlungseinheit 1 kann die Ultrafiltrationsrate eingestellt werden. Die Druckverhältnisse können mit der Drossel 16 oder der Ultrafiltrationspumpe 15 verändert werden, die von der Steuereinheit 17 angesteuert werden können. Dabei dient die Ultrafiltrationspumpe 15 als Mittel zur Erhöhung der transmembranen Druckdifferenz durch relative Absenkung des Drucks in der Dialysierflüssigkeitskammer gegenüber dem Druck in der Blutkammer. Die transmembrane Druckdifferenz kann aber auch durch eine Steigerung des Drucks in der Blutkammer erhöht werden.

Als vorteilhaft kann sich erweisen, wenn die Steuereinheit 17 die Impellerpumpe 3 und/oder die Dialysierflüssigkeitspumpe 19 in einem Pulsmodus betreibt, um einen nicht kontinuierlichen Blut- bzw. Dialysierflüssigkeitsfluss zu erzeugen. Dies kann beispielsweise durch fortlaufendes Ein- und Ausschalten einer der beiden oder beider Pumpen 3, 15 oder durch fortlaufendes Erhöhen und Verringern der Förderraten einer oder beider Pumpen erreicht werden. Dieser Pulsbetrieb ist alternativ auch durch Betätigung von in den Leitungen angeordneten Ventilen möglich.

Die erfindungsgemäße extrakorporale Dialysevorrichtung zeichnet sich durch einen kompakten Aufbau aus, da "Blutpumpe" und "Dialysator" der Dialysevorrichtung zu einer Einheit zusammengefasst sind, die aufgrund der verhältnismäßig geringen Abmessungen der Dialysatormembran sich in einem relativ kleinen Gehäuse unterbringen lässt.

## Patentansprüche

1. Blutbehandlungseinheit für eine extrakorporale Blutbehandlungsvorrichtung mit
einem eine erste und eine zweite Kammer (2A, 2B) aufweisenden Gehäuse (2), das eine Blutpumpe (3) und eine semipermeable Membran (4) aufnimmt,
wobei
die Blutpumpe eine Impellerpumpe (3) ist, die einen in dem Gehäuse (2) um eine Achse (3B) drehbar gelagerten Impeller (3A) aufweist, der von einer das Gehäuse (2) in die erste und zweite Kammer (2A, 2B) unterteilenden Trennwand umschlossen wird, und
die semipermeable Membran (4) zumindest einen Teil der Trennwand bildet, so dass über die Membran ein Stoffaustausch zwischen Blut in der ersten Kammer (2A) und Dialysierflüssigkeit in der zweiten Kammer (2B) stattfinden kann,
**dadurch gekennzeichnet, dass**
das Gehäuse (2) einen von der ersten Kammer (2A) abgehenden Auslasskanal (6) zum Abführen von Blut aufweist, der sich in radialer Richtung erstreckt, und
die semipermeable Membran (4) von dem Auslasskanal (6) zum Abführen von Blut in radialer Richtung durchdrungen wird.

2. Blutbehandlungseinheit nach Anspruch 1, **dadurch gekennzeichnet, dass** das Gehäuse (2) einen zu der ersten Kammer (2A) führenden Einlasskanal (5) zum Zuführen von Blut aufweist, der sich in Richtung der Drehachse (3B) des Impellers (3) erstreckt.

3. Blutbehandlungseinheit nach Anspruch 1 oder 2, **dadurch gekennzeichnet, dass** das Gehäuse (2) einen zu der zweiten Kammer (2B) führenden Einlasskanal (7) zum Zuführen von Dialysierflüssigkeit aufweist.

4. Blutbehandlungseinheit nach einem der Ansprüche 1 bis 3, **dadurch gekennzeichnet, dass** das Gehäuse (2) einen von der zweiten Kammer (2B) abgehenden Auslasskanal (8) zum Abführen von Dialysierflüssigkeit aufweist.

5. Blutbehandlungseinheit nach einem der Ansprüche 1 bis 4, **dadurch gekennzeichnet, dass** die zweite Kammer (2B) des Gehäuses (2) als Ringraum ausgebildet ist.

6. Blutbehandlungseinheit nach Anspruch 5, **dadurch gekennzeichnet, dass** die zweite Kammer (2B) als ein die erste Kammer (2A) vollständig umschließender Raum ausgebildet ist.

7. Blutbehandlungseinheit nach einem der Ansprüche 1 bis 6, **dadurch gekennzeichnet, dass** die semipermeable Membran (4) an der Oberfläche, die der ersten Kammer (2A) zugewandt ist, mit Rillen versehen ist.

8. Extrakorporale Blutbehandlungsvorrichtung mit einer Blutbehandlungseinheit (1) nach einem der Ansprüche 1 bis 7, **dadurch gekennzeichnet, dass** die extrakorporale Dialysevorrichtung eine arterielle Blutleitung (9) zum Zuführen von Blut zu der ersten Kammer (2A) und eine venöse Blutleitung (10) zum Abführen von Blut aus der ersten Kammer (2A) der Blutbehandlungseinheit (1) aufweist.

9. Extrakorporale Blutbehandlungsvorrichtung nach Anspruch 8, **dadurch gekennzeichnet, dass** die Blutbehandlungsvorrichtung eine Dialysierflüssigkeitszuführleitung (12) zum Zuführen von Dialysierflüssigkeit zu der zweiten Kammer (2B) und eine Dialysierflüssigkeitsabführleitung (13) zum Abführen von Dialysierflüssigkeit aus der zweiten Kammer (2B) der Blutbehandlungseinheit (1) aufweist.

10. Extrakorporale Blutbehandlungsvorrichtung nach Anspruch 8 oder 9, **dadurch gekennzeichnet, dass** in der arteriellen Blutleitung (9) Mittel (16) zum Drosseln des Blutflusses angeordnet sind.

11. Extrakorporale Blutbehandlungsvorrichtung nach Anspruch 9 oder 10, **dadurch gekennzeichnet, dass** in der Dialysierflüssigkeitszuführleitung (12) eine Dialysierflüssigkeitspumpe (19) angeordnet ist.

12. Extrakorporale Blutbehandlungsvorrichtung nach Anspruch 11, **dadurch gekennzeichnet, dass** die Blutbehandlungsvorrichtung eine Steuereinheit (17) aufweist, die derart ausgebildet ist, dass die Blutpumpe (3) und/oder die Dialysierflüssigkeitspumpe (19) in einem Pulsmodus betrieben wird, in dem die Blutpumpe (3) bzw. Dialysierflüssigkeitspumpe (19) fortlaufend ein- und ausgeschaltet wird oder die Drehzahl der Blutpumpe bzw. Dialysierflüssigkeitspumpe fortlaufend erhöht und verringert wird.

## Claims

1. Blood treatment unit for an extra-corporeal blood treatment apparatus, having a housing (2) which has a first and a second chamber (2A, 2B) and which holds a blood pump (3) and a semi-permeable membrane (4), the blood pump being an impeller pump (3) which has an impeller (3A) which is mounted in the housing (2) to be rotatable on an axis (3B) and which is surrounded by a partition which divides the housing (2) into the first and second chambers (2A, 2B), and the semi-permeable membrane (4) forming at least a part of the partition, thus enabling an exchange of matter to take place via the membrane between blood in the first chamber (2A) and dialysis fluid in the second chamber (2B), **characterised in that** the housing (2) has an outlet duct (6) running away from the first chamber (2A) for the outfeed of blood, which duct extends in the radial direction, and the outlet duct (6) for the outfeed of blood passes through the semi-permeable membrane (4) in the radial direction.

2. Blood treatment unit according to claim 1, **characterised in that** the housing (2) has an inlet duct (5) for the infeed of blood which runs to the first chamber (2A) and which extends in the direction defined by the axis of rotation (3B) of the impeller (3).

3. Blood treatment unit according to claim 1 or 2, **characterised in that** the housing (2) has an inlet duct (7) for the infeed of dialysis fluid which runs to the second chamber (2B).

4. Blood treatment unit according to one of claims 1 to 3, **characterised in that** the housing (2) has an outlet duct (8) running away from the second chamber (2B) for the outfeed of dialysis fluid.

5. Blood treatment unit according to one of claims 1 to 4, **characterised in that** the second chamber (2B) of the housing (2) takes the form of an annular space.

6. Blood treatment unit according to claim 5, **characterised in that** the second chamber (2B) takes the form of a space which completely surrounds the first chamber (2A).

7. Blood treatment unit according to one of claims 1 to 6, **characterised in that** the semi-permeable membrane (4) is provided with grooves on the surface adjacent the first chamber (2A).

8. Extra-corporeal blood treatment apparatus having a blood treatment unit (1) according to one of claims 1 to 7, **characterised in that** the extra-corporeal dialysis apparatus has an arterial blood line (9) for feeding blood into the first chamber (2A) of the blood treatment unit (1) and a venous blood line (10) for feeding blood out of the first chamber (2A) thereof.

9. Extra-corporeal blood treatment apparatus according to claim 8, **characterised in that** the blood treatment apparatus has an infeed line (12) for dialysis fluid for feeding dialysis fluid to the second chamber (2B) of the blood treatment unit (1) and an outfeed line (13) for dialysis fluid for feeding dialysis fluid out of the second chamber (2B) thereof.

10. Extra-corporeal blood treatment apparatus according to claim 8 or 9, **characterised in that** means (16) for throttling the flow of blood are arranged in the arterial blood line (9).

11. Extra-corporeal blood treatment apparatus according to claim 9 or 10, **characterised in that** a dialysis-fluid pump (19) is arranged in the dialysis-fluid infeed line (12).

12. Extra-corporeal blood treatment apparatus according to claim 11, **characterised in that** the blood treatment apparatus has a control unit (17) which is so designed that the blood pump (3) and/or the dialysis-fluid pump (19) is operated in a pulsed mode in which the blood pump (3) or the dialysis-fluid pump (19) is switched on and off continuously or the speed of revolution of the blood pump or dialysis-fluid pump is increased and decreased continuously.

## Revendications

1. Unité de traitement de sang destinée à un dispositif extracorporel de traitement de sang dotée d'un logement (2) présentant une première et une deuxième chambre (2A, 2B), qui reçoit une pompe à sang (3) et une membrane semi-perméable (4),
la pompe à sang étant une pompe centrifuge (3), qui présente un impulseur (3A) monté de manière à pouvoir pivoter autour d'un axe (3B) dans le logement (2), qui est entouré d'une cloison subdivisant le logement (2) en la première et la deuxième chambre (2A, 2B), et
la membrane semi-perméable (4) formant au moins une partie de la cloison, de sorte qu'un échange de substances, entre du sang dans la première chambre (2A) et du liquide de dialyse dans la deuxième chambre (2B) puisse avoir lieu via la membrane,
**caractérisée en ce que**
le logement (2) présente un canal d'évacuation (6) partant de la première chambre (2A) pour évacuer du sang, qui s'étend dans le sens radial, et
la membrane semi-perméable (4) est traversée par le canal d'évacuation (6) pour évacuer du sang dans le sens radial.

2. Unité de traitement de sang selon la revendication 1, **caractérisée en ce que** le logement (2) présente un canal d'admission (5) conduisant à la première chambre (2A) pour amener du sang, qui s'étend dans le sens de l'axe de rotation (3B) de l'impulseur (3).

3. Unité de traitement de sang selon la revendication 1 ou 2, **caractérisée en ce que** le logement (2) présente un canal d'admission (7) conduisant à la deuxième chambre (2B) pour amener du liquide de dialyse.

4. Unité de traitement de sang selon l'une des revendications 1 à 3, **caractérisée en ce que** le logement (2) présente un canal d'évacuation (8) partant de la deuxième chambre (2B) pour évacuer du liquide de dialyse.

5. Unité de traitement de sang selon l'une des revendications 1 à 4, **caractérisée en ce que** la deuxième chambre (2B) du logement (2) est façonnée comme un espace annulaire.

6. Unité de traitement de sang selon la revendication 5, **caractérisée en ce que** la deuxième chambre (2B) est façonnée comme un espace entourant complètement la première chambre (2A).

7. Unité de traitement de sang selon l'une des revendications 1 à 6, **caractérisée en ce que** la membrane semi-perméable (4) sur la surface qui est orientée vers la première chambre (2A) est munie de rainures.

8. Dispositif extracorporel de traitement de sang doté d'une unité de traitement de sang (1) selon l'une des revendications 1 à 7, **caractérisé en ce que** le dispositif extracorporel de dialyse présente une ligne à sang artérielle (9) pour amener du sang vers la première chambre (2A) et une ligne à sang veineuse (10) pour évacuer du sang de la première chambre (2A) de l'unité de traitement de sang (1).

9. Dispositif extracorporel de traitement de sang selon la revendication 8, **caractérisé en ce que** le dispositif de traitement de sang présente une ligne d'alimentation en liquide de dialyse (12) pour amener du liquide de dialyse à la deuxième chambre (2B) et une ligne d'évacuation de liquide de dialyse (13) pour évacuer du liquide de dialyse de la deuxième chambre (2B) de l'unité de traitement de sang (1).

10. Dispositif extracorporel de traitement de sang selon la revendication 8 ou 9, **caractérisé en ce qu'**un moyen (16) est disposé dans la ligne à sang artérielle (9) pour réduire le débit sanguin.

11. Dispositif extracorporel de traitement de sang selon la revendication 9 ou 10, **caractérisé en ce qu'**une pompe à liquide de dialyse (19) est disposée dans la ligne d'alimentation en liquide de dialyse (12).

12. Dispositif extracorporel de traitement de sang selon la revendication 11, **caractérisé en ce que** le dispositif de traitement de sang présente une unité de commande (17), qui est façonnée de telle manière que la pompe à sang (3) et/ou la pompe à liquide de dialyse (19) fonctionnent en mode impulsionnel, dans lequel mode la pompe à sang (3) ou la pompe à liquide de dialyse (19) est constamment allumée et éteinte ou la vitesse de rotation de la pompe à sang ou de la pompe à liquide de dialyse est constamment augmentée ou diminuée.
